# EUROPÄISCHE PATENTSCHRIFT

(11) **EP 2 990 183 B1**
(45) Veröffentlichungstag und Bekanntmachung des Hinweises auf die Patenterteilung: **02.10.2019**
(21) Anmeldenummer: 15180183.4
(22) Anmeldetag: 07.08.2015
(51) Int. Cl.: B29C 65/48, F16L 47/02, B29C 65/14, B29C 65/54, B29C 65/00, B29L 23/00, A61M 39/12

(54) **VERFAHREN ZUR HERSTELLUNG EINER FLUIDDICHTEN VERBINDUNG ZWISCHEN MINDESTENS ZWEI FLUIDFÜHRENDEN SILIKON-SCHLAUCHKOMPONENTEN SOWIE FLUIDFÜHRUNGS-BAUGRUPPE, HERGESTELLT NACH DIESEM VERFAHREN**
PROCESS FOR THE MANUFACTURE OF AN IMPERMEABLE CONNECTION BETWEEN AT LEAST TWO FLUID CARRYING SILICONE HOSE COMPONENTS AND A FLUID CARRYING ASSEMBLY MANUFACTURED ACCORDING TO SAID PROCESS
PROCEDE DE FABRICATION D'UNE LIAISON ETANCHE AUX FLUIDES ENTRE AU MOINS DEUX COMPOSANTS DE TUYAU EN SILICONE TRANSPORTANT DES FLUIDES ET COMPOSANT CONDUCTEUR DE FLUIDE FABRIQUE SELON LEDIT PROCEDE

(30) Priorität: 18.08.2014 DE 102014216329
(43) Veröffentlichungstag der Anmeldung: 02.03.2016
(73) Patentinhaber: RAUMEDIC AG, 95213 Münchberg (DE)
(72) Erfinder: Gläsel, Björn, 95158 Kirchenlamitz (DE); Prescher, Jörg, 97729 Ramsthal (DE); Wunderlich, Axel, 08626 Ebmath (DE); Reichenberger, Robert, 95632 Wunsiedel (DE)
(74) Vertreter: Rau, Schneck & Hübner Patentanwälte Rechtsanwälte PartGmbB

(56) Entgegenhaltungen:
- WO-A2-2007/048072

## Beschreibung

Die Erfindung betrifft ein Verfahren zur Herstellung einer fluiddichten und insbesondere mediendichten Verbindung zwischen mindestens zwei fluidführenden Silikon-Schlauchkomponenten. Ferner betrifft die Erfindung eine Fluidführungs-Baugruppe mit mindestens zwei fluidführenden Silikon-Schlauchkomponenten und mindestens einer Silikon-Konnektorkomponente, zwischen denen eine fluidführende Verbindung über ein derartiges Verfahren hergestellt ist.

Eine Fluidführungs-Baugruppe mit mehreren Schlauchkomponenten und mindestens einer Konnektorkomponente ist bekannt aus der WO 2012/163819 A2, aus der Beispiele für die Konnektorkomponenten-Variante "T-Konnektor", "L-Konnektor", "Y-Konnektor" und "gerader Konnektor" bekannt sind. Weitere Fluidführungs-Baugruppen sind bekannt aus der WO 2009/117059 A2 und aus der WO 2007/048072 A2.

Es ist eine Aufgabe der vorliegenden Erfindung, eine einerseits sichere und andererseits eine Automatisierung ermöglichende Verbindungstechnik zur Herstellung einer entsprechenden fluiddichten und insbesondere mediendichten Verbindung und damit einer Fluidführungs-Baugruppe zu schaffen.

Diese Aufgabe ist erfindungsgemäß gelöst durch ein Herstellungsverfahren mit den in Anspruch 1 angegebenen Merkmalen.

Beim erfindungsgemäßen Herstellungsverfahren wird eine Silikon/Silikon-Verbindung hergestellt. Nach dem polymeren Nachvernetzen ergibt sich eine innige Materialverbindung der über die Silikonmasse miteinander verbundenen Silikon-Komponenten, die die Charakteristiken einer einstückigen Verbindung aufweist. Bei einem In-Kontakt-Bringen vor dem Aufbringen der Silikonmasse lässt sich vermeiden, dass die Silikonmasse in einen Fluidführungs-Kanal der hergestellten Fluidführungs-Baugruppe eindringt. Eine Silikon-Konnektorkomponente kann mit zwei bis sechs Silikon-Schlauchkomponenten, je nach Ausführung der Konnektorkomponente, verbunden sein. Ein Verbindungsbereich kann der Verbindung der Silikon-Konnektorkomponente mit genau einer Silikon-Schlauchkomponente oder auch der Verbindung genau einer Konnektorkomponente mit einer Mehrzahl von Schlauchkomponenten dienen. Die Konnektorkomponente und/oder die Schlauchkomponenten können als Spritzguss-Bauteile gefertigt sein. Die hergestellte fluiddichte Verbindung kann auch für andere Medien dicht sein, insbesondere für Feststoffe. Das polymere Nachvernetzen des Verbindungsbereiches kann durch Tempern geschehen. Durch Tempern der Verbindungsstellen werden die Verbindungsstellen über einen bestimmten Zeitraum erwärmt, ohne die Silikonmasse zu verformen, wodurch die Silikonmasse aushärtet. Das polymere Nachvernetzen kann alternativ oder zusätzlich auch über andere Einflussgrößen initiiert werden, beispielsweise über eine Bestrahlung mit UV-Licht und/oder über eine gezielte Luftfeuchtigkeitsänderung, insbesondere über eine gezielte Luftfeuchtigkeitserhöhung. Das Tempern kann in einem Zeitraum zwischen 10 Minuten und 5 Stunden erfolgen, insbesondere in einem Zeitraum zwischen 30 Minuten und 2 Stunden. Ein Temperaturbereich beim Tempern kann im Bereich zwischen 80 °C und 200 °C, insbesondere im Bereich zwischen 150 °C und 200 °C liegen.

Beim Aufbringen der Silikonmasse auf den Verbindungsbereich kann auf diese eine definierte Zugkraft ausgeübt werden. Dies führt zu einer internen Zug-Vorspannung (stretch) der aufgebrachten Silikonmasse, was ein nachfolgendes Nachvernetzen begünstigt.

Ein Einbringen der Silikonmasse in einen nach außen verdeckten Aufnahmeraum des Verbindungsbereiches vermeidet, dass die ausgehärtete Silikonmasse bei einem hergestellten Produkt sichtbar ist. Eine einheitliche Optik kann so gewährleistet werden.

Ein Vorhärten der Silikonmasse nach Anspruch 2 kann durch eine Wärmebehandlung und insbesondere durch eine Infrarot-Bestrahlung erfolgen. Das Vorhärten kann zu einem Vernetzen der Silikonmasse führen. Das Vorhärten ergibt eine Vorpositionierung beziehungsweise Fixierung der Silikonmasse vor dem eigentlichen polymeren Nachvernetzen. Dies erleichtert das Handling des Verbindungsbereiches während des Herstellungsverfahrens.

Ein Ineinanderschieben der Komponenten nach Anspruch 3 führt zu einer sicheren Verbindung der Komponenten.

Ein Vorzentrieren nach Anspruch 4 vermeidet insbesondere eine unerwünschte radiale Relativverlagerung der zu verbindenden Komponenten und verhindert, dass unerwünschte Toträume entstehen. Zudem kann die Baugruppe mit der mindestens einen Konnektorkomponente und den Schlauchkomponenten vorgefertigt und in einem nachfolgenden Schritt mit Hilfe der Silikonmasse endgültig verbunden werden.

Bei einem Verfahren nach Anspruch 5 wird die Silikonmasse über vordefinierte Einbringkanäle in den Aufnahmeraum eingebracht. Der Aufnahmeraum und/oder der mindestens eine Einbringkanal können so gestaltet sein, dass eine exakt vorgegebene Menge an Silikonmasse für den Verbindungsprozess eingesetzt werden kann. Eine Sichtblende zum Verdecken des Aufnahmeraums kann die Optik des Verbindungsbereiches nochmals verbessern.

Ein Aufweiten einer Sichtblende nach Anspruch 6 ermöglicht ein Einbringen der fließfähigen Silikonmasse über einen von der Sichtblende zunächst freigegebenen Einbringkanal. Das Aufweiten der Sichtblende kann mechanisch oder chemisch erfolgen. Das Aufweiten kann vor einem Ineinanderschieben der Komponenten erfolgen.

Die Vorteile einer Fluidführungs-Baugruppe nach Anspruch 7 entsprechen denen, die vorstehend unter Bezugnahme auf das Herstellungsverfahren bereits erläutert wurden.

Komponenten aus der gleichen Silikon-Materialzusammensetzung nach Anspruch 8 vereinfachen insbesondere eine medizintechnische Zulassung der Fluidführungs-Baugruppe. Auch die fließfähige Silikonmasse kann aus der gleichen Silikon-Materialzusammensetzung bestehen wie die Silikon-Konnektorkomponente und/oder wie die Silikon-Schlauchkomponenten.

Die Vorteile der Sichtblende nach den Ansprüchen 9 beziehungsweise 10 wurden im Zusammenhang mit dem Herstellungsverfahren bereits erläutert.

Je nach Ausführung der Fluidführungs-Baugruppe kann genau ein Einbringkanal nach Anspruch 11 oder es können auch mehrere, zum Beispiel zwei bis fünf Einbringkanäle vorgesehen sein. Der mindestens eine Einbringkanal kann radial zu einer Schlauchachse, zu einer Längsachse der Komponenten verlaufen. Mehrere Einbringkanäle können in Umfangsrichtung um diese Schlauchachse gleich verteilt angeordnet sein. Der mindestens eine Einbringkanal kann im Konnektor ausgeführt sein.

Über einen Zentrierabsatz nach Anspruch 12 kann ein Vorzentrieren der Schlauchkomponenten relativ zur Konnektorkomponente gewährleistet werden.

Ausführungsbeispiele der Erfindung werden nachfolgend anhand der Zeichnung näher erläutert. In dieser zeigen:
Fig. 1 und 2 jeweils einen Längsschnitt durch einen Ausschnitt einer Fluidführungs-Baugruppe, wobei ein nicht erfindungsgemäßer Verbindungsbereich zwischen einer Silikon-Konnektorkomponente und einer Silikon-Schlauchkomponente dargestellt ist; und
Fig. 3 bis 6 jeweils eine erfindungsgemäße Ausführungsform eines Verbindungsbereiches einer Fluidführungs-Baugruppe.

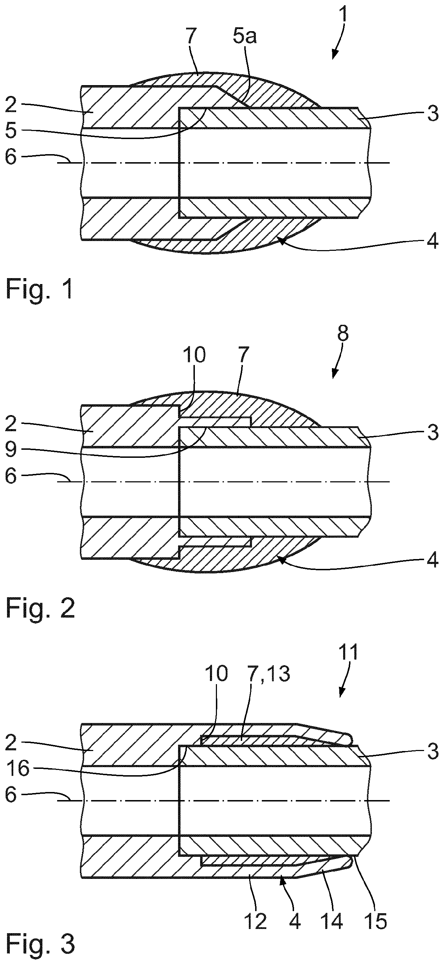

Fig. 1 zeigt zur Erläuterung der vorliegenden Erfindung einen nicht erfindungsgemäßen Verbindungsbereich einer Fluidführungs-Baugruppe 1 im Längsschnitt. Die Fluidführungs-Baugruppe 1 kommt in medizinischen Anwendungen zum Einsatz. Beispiele für derartige Anwendungen sind angegeben in der WO 2012/163819 A2 und den dort angegebenen Referenzen.

Die Fluidführungs-Baugruppe 1 hat neben mindestens einer Silikon-Konnektorkomponente 2 mehrere Silikon-Schlauchkomponenten, von denen in der Fig. 1 genau eine Silikon-Schlauchkomponente 3 dargestellt ist. Über die Konnektorkomponente 2 sind die Schlauchkomponenten 3 miteinander verbunden. Die Konnektorkomponente 2 kann als T-Konnektor, als L-Konnektor, als Y-Konnektor oder auch als gerader Konnektor ausgeführt sein. Über eine Konnektorkomponente 2 können also zwei oder drei Schlauchkomponenten 3 miteinander verbunden sein. Grundsätzlich können auch noch mehr Schlauchkomponenten über eine Konnektorkomponente verbunden sein, beispielsweise vier, fünf oder sechs Schlauchkomponenten. Die Konnektorkomponente 2 einerseits und die Schlauchkomponenten 3 andererseits können aus der gleichen Silikon-Materialzusammensetzung bestehen.

Im Verbindungsbereich 4 der Fluidführungs-Baugruppe 1, also dort, wo die Schlauchkomponente 3 mit der Konnektorkomponente 2 verbunden ist, hat die Konnektorkomponente 2 einen Zentrierabsatz 5. Der Zentrierabsatz 5 bildet eine in Bezug auf eine Längsachse 6 des Verbindungsbereichs 4 zentrierte Aufnahme der Konnektorkomponente 2, in die die Schlauchkomponente 3 eingeschoben ist. Eine der Schlauchkomponente 3 zugewandte, führende Stirnwand 5a der Konnektorkomponente 2 ist sich zu einer äußeren Umfangswand der Schlauchkomponente 3 hin konisch verjüngend ausgeführt. Dies begünstigt einen dichten Abschluss zwischen der Konnektorkomponente 2 und der Schlauchkomponente 3 im Verbindungsbereich 4.

Zur fluiddichten Verbindung der Konnektorkomponente 2 mit der Schlauchkomponente 3 dient eine ausgehärtete Silikonmasse 7, die zuvor als fließfähige Rohmasse auf den Verbindungsbereich 4 aufgebracht wurde. Zum Aushärten der Silikonmasse 7 wurde nach dem Aufbringen der Verbindungsbereich 4 nachvernetzt.

Zur Herstellung der fluiddichten Verbindung im Verbindungsbereich 4 kann die Schlauchkomponente 3 zuerst in die Konnektorkomponente 2 eingeschoben und anschließend die noch fließfähige Silikonmasse 7 aufgebracht werden oder es kann umgekehrt zuerst die Silikonmasse vorbereitend auf der Konnektorkomponente 2 und/oder der Schlauchkomponente 3 im Verbindungsbereich 4 aufgebracht und anschließend die Schlauchkomponente 3 in die Konnektorkomponente 2 eingeschoben werden.

Bei einer nicht dargestellten Ausführung kann auch die Schlauchkomponente den Zentrierabsatz 5 aufweisen, in den in diesem Fall die Konnektorkomponente eingeschoben ist.

Bei der Ausführung nach Fig. 1 dient der Verbindungsbereich 4 zur Verbindung der Konnektorkomponente 2 mit genau einer Schlauchkomponente 3. Bei einer nicht dargestellten Ausführungsform kann der Verbindungsbereich 4 auch der Verbindung der Konnektorkomponente mit mehreren Schlauchkomponenten dienen.

Bei der Silikonmasse 7 kann es sich um hochtemperaturvernetztes (HTV) Silikon, um Silikonkautschuk (Liquid Silicon Rubber, LSR), um einen Silikonkleber oder auch um ein anderweitiges Silikon handeln.

Zur Herstellung der fluiddichten Verbindung zwischen mindestens zwei fluidführenden Silikon-Schlauchkomponenten nach Art der Schlauchkomponente 3 werden zunächst die Silikon-Schlauchkomponenten und mindestens eine Silikon-Konnektorkomponente, zum Beispiel nach Art der Konnektorkomponente 2, bereitgestellt. Anschließend wir die fließfähige Silikonmasse 7 auf den mindestens einen Verbindungsbereich 4 aufgebracht, an dem die Silikon-Schlauchkomponente 3 an der Silikon-Konnektorkomponente 2 anliegt. Sodann wird der mindestens eine Verbindungsbereich 4 zum Aushärten der Silikonmasse 7 nachvernetzt.

Die fließfähige Silikonmasse 7 kann vor dem Aufbringen auf den Verbindungsbereich 4 noch vorbereitet werden. Hierzu kann zunächst eine Roh-Silikonmasse gewalzt und aus dieser gewalzten Roh-Silikonmasse ein Vorformling hergestellt werden, beispielsweise ein Strang mit vorgegebenem Durchmesser. Ein derartiger Strang kann durch Extrusion hergestellt werden. Alternativ kann anstelle eines Strangs ein Vorformling in Form eines Bandes oder eines Profils insbesondere durch Extrusion hergestellt werden. Insbesondere ein strangförmiger Vorformling der Roh-Silikonmasse kann zwischen zwei Folien flach gewalzt werden. Bei den Folien kann es sich um geriefte oder um genoppte Folien handeln, was eine Entnahme des Vorformlings aus den diesen umgebenden Folien nach dem Walzen erleichtert. Die zwischen den Folien gewalzte Silikonmasse ist nach dem Entnehmen zum Aufbringen auf den Verbindungsbereich 4 geeignet.

Beim Aufbringen der fließfähigen Silikonmasse 7 auf den Verbindungsbereich kann diese gedehnt werden. Dies wird erreicht, indem beim Aufbringen eine Zugspannung auf die Silikonmasse ausgeübt wird.

Vor dem polymeren Nachvernetzen, also insbesondere vor einem Tempern, kann ein Vorhärten der Silikonmasse 7 erfolgen. Dieses Vorhärten kann durch Bestrahlen der aufgebrachten Silikonmasse mit Infrarotlicht geschehen. Das Vorhärten dient zum Vernetzen der Silikonmasse 7 und/oder zur Vorpositionierung beziehungsweise Fixierung der Silikonmasse 7 vor dem eigentlichen Nachvernetzen.

Zum In-Kontakt-Bringen der Schlauchkomponente 3 mit der Konnektorkomponente 2, was vor oder nach dem Aufbringen der Silikonmasse 7 geschehen kann, wird bei der Ausführung nach Fig. 1 die Schlauchkomponente 3 in den Zentrierabsatz 5 der Konnektorkomponente 2 eingeschoben. Bei der Ausführung nach Fig. 1 wird die Silikonmasse außen auf den Verbindungsbereich 4 aufgebracht. Die Silikonmasse 7 dichtet den Verbindungsbereich vollumfänglich um die Längsachse 6 herum ab.

Anhand der Fig. 2 bis 6 werden nachfolgend weitere Ausführungen von Fluidführungs-Baugruppen erläutert. Komponenten, die denjenigen entsprechen, die unter Bezugnahme auf andere Figuren bereits erläutert wurden, tragen die gleichen Bezugsziffern und werden nicht nochmals im Einzelnen diskutiert.

Die nicht erfindungsgemäße Fluidführungs-Baugruppe 8 nach Fig. 2 hat einen Zentrierabsatz 9 in Form einer Zentrierhülse, die Teil der Konnektorkomponente 2 ist und an eine Stirnwand 10 der Konnektorkomponente 2 angeformt ist. Auf Höhe des Zentrierabsatzes 9 verringert sich auch ein Außenumfang der Konnektorkomponente 2 stufenförmig. Im Verbindungsbereich 4 der Fluidführungs-Baugruppe 8 liegen somit mehrere rechtwinklige Stufen-Übergänge vor, was eine Haftung der Silikonmasse 7 begünstigt.

Bei der erfindungsgemäßen Fluidführungs-Baugruppe 11 nach Fig. 3 hat die Konnektorkomponente 2 eine Sichtblende 12 zum Verdecken eines Aufnahmeraums 13 des Verbindungsbereichs 4, an dem die Schlauchkomponente 3 an der Konnektorkomponente 2 anliegt. Der Aufnahmeraum 13 ist als die Schlauchkomponente 3 außen umgebender Ringraum ausgeführt, der sich vollumfänglich um die Längsachse 6 erstreckt.

Die Sichtblende 12 ist als Sichthülse ausgeführt, deren Außenumfang zunächst dem Außenumfang der Konnektorkomponente 2 entspricht, wobei sich die Sichtblende 12 im Bereich eines freien Endes 14 konisch verjüngt, sodass die Sichtblende 12 am freien Ende 14 direkt an einer äußeren Mantelwand 15 der Schlauchkomponente 3 anliegt. Der Aufnahmeraum 13 ist somit allseitig geschlossen. Die Silikonmasse 7 liegt bei der Ausführung nach Fig. 3 im Aufnahmeraum 13 vor und füllt diesen aus. Zugeführt wird die Silikonmasse 7 dem Aufnahmeraum 13 entweder vor dem Einschieben der Schlauchkomponente 3 in die Konnektorkomponente 2 oder nach dem Einschieben.

Auch bei der Ausführung nach Fig. 3 hat die Konnektorkomponente 2 einen Zentrierabsatz 16 für die Schlauchkomponente 3, der als Umfangsstufe in der Stirnwand 10 der Konnektorkomponente 2 ausgeführt ist.

Beim Aufbringen der Silikonmasse 7 nach dem Einschieben der Schlauchkomponente 3 in Konnektorkomponente 2 wird die Silikonmasse 7 zum Beispiel zwischen dem freien Ende 14 und der Mantelwand 15 eingespritzt. Ein Einspritzen kann alternativ auch über einen nicht näher dargestellten Einbringkanal erfolgen, der eine Fluidverbindung zwischen dem Aufnahmeraum 13 und einer äußeren Umgebung der Fluidführungs-Baugruppe 11 darstellt. Der Einbringkanal kann radial und/oder axial in Bezug auf die Längsachse 6 verlaufen. Es kann eine Mehrzahl von Einbringkanälen vorhanden sein. Es können beispielsweise zwei, drei, vier, fünf oder noch mehr Einbringkanäle vorhanden sein. Die Einbringkanäle können in Umfangsrichtung um die Längsachse gleich verteilt angeordnet sein.

Beim Herstellen der fluiddichten Verbindung wird die Silikonmasse 7 beim Aufbringen in den nach außen verdeckten Aufnahmeraum 13 des Verbindungsbereiches 4 eingebracht.

Der mindestens eine Einbringkanal kann in der Konnektorkomponente 2 ausgeführt sein.

Alternativ oder zusätzlich kann die äußere Konnektorkomponente 2 im Verbindungsbereich 4 mit Hilfe einer Aufspreizeinrichtung aufgespreizt werden, sodass die innere Schlauchkomponente 3 mit der vorab aufgebrachten Silikonmasse 7 in den zunächst aufgespreizten Verbindungsbereich 7 eingeschoben werden kann, ohne dass hierbei eine Außenseite der Silikonmasse 7 mit einer Innenwand der Konnektorkomponente 2 im Verbindungsbereich 4 in Kontakt kommt. Nach dem Einschieben der Schlauchkomponente 3 in die Konnektorkomponente kann das Aufspreizen beendet werden, so dass die Silikonmasse 7 radial innen an der Schlauchkomponente 3 und radial außen an der Konnektorkomponente im Verbindungsbereich 4 anliegt. Es kann dann das polymere Nachvernetzen zum Aushärten der Silikonmasse 7 erfolgen. Hierbei kann der Verbindungsbereich beispielsweise für die Dauer von zwei Stunden bei 200 °C getempert werden.

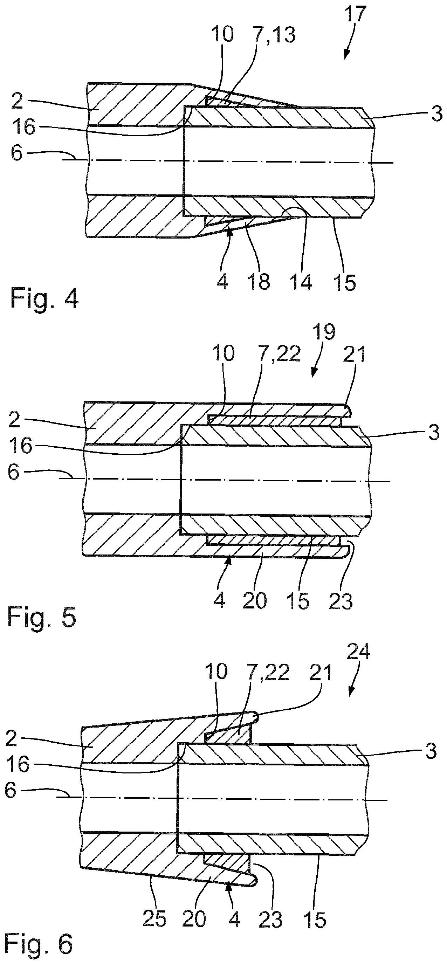

Bei der Ausführung nach der Fluidführungs-Baugruppe 17 nach Fig. 4 ist eine Sichtblende 18, deren Funktion ansonsten derjenigen der Sichtblende 12 nach Fig. 3 entspricht, konisch sich verjüngend ausgeführt. Die Sichtblende 18 verläuft wiederum ausgehend von der Stirnwand 10 und liegt über ihr freies Ende 14 flächig an der Mantelwand 15 der Schlauchkomponente 3 an. Bei der Ausführung nach Fig. 4 hat der Aufnahmeraum 13 einen dreieckigen Ringquerschnitt.

Vor einem In-Kontakt-Bringen der Schlauchkomponente 3 an der Konnektorkomponente 2 kann die Sichtblende 12 beziehungsweise 18 aufgeweitet werden.

Fig. 5 zeigt eine weitere Ausführung der Fluidführungs-Baugruppe 19. Eine Sichtblende 20 der Fluidführungs-Baugruppe 19 unterscheidet sich z.B. von der Sichtblende 12 der Ausführung nach Fig. 3 dadurch, dass die Sichtblende 20 an ihrem freien Ende 21 nicht an der äußeren Mantelwand 15 der Schlauchkomponente 3 anliegt. Ein Aufnahmeraum 22 für die Silikonmasse 7 ist bei der Ausführung nach Fig.5 also nicht allseitig abgeschlossen, sondern es verbleibt eine Ringöffnung 23 zwischen dem freien Ende 21 und der Mantelwand 15, über die der Aufnahmeraum 22 von außen her zugänglich ist.

Fig. 6 zeigt eine weitere Ausführung der Fluidführungs-Baugruppe 24 mit einer Sichtblende 20 und einem von außen zugänglichen Aufnahmeraum 22. Im Unterschied zur Ausführung nach Fig. 5 erweitert sich der Aufnahmeraum 22 hin zur Ringöffnung 23 konisch. Hiermit korrespondiert eine konische Erweiterung einer äußeren Mantelwand 25 der Konnektorkomponente 2, die im Verbindungsbereich 4 in die Sichtblende 20 übergeht.

## Patentansprüche

1. Verfahren zur Herstellung einer fluiddichten Verbindung zwischen mindestens zwei fluidführenden Silikon-Schlauchkomponenten (3) mit folgenden Schritten:
- Bereitstellen der Silikon-Schlauchkomponenten (3) und einer Silikon-Konnektorkomponente (2),
- Aufbringen einer fließfähigen Silikonmasse (7) auf mindestens einen Verbindungsbereich (4), an dem die Silikon-Schlauchkomponenten (3) an der Silikon-Konnektorkomponente (2) anliegen,
- Polymeres Nachvernetzen des mindestens einen Verbindungsbereiches (4) zum Aushärten der Silikonmasse (7),
**dadurch gekennzeichnet, dass**
- ein In-Kontakt-Bringen der Silikon-Schlauchkomponenten (3) an der Silikon-Konnektorkomponente (2) vor dem Aufbringen der fließfähigen Silikonmasse (7) erfolgt und
- die Silikonmasse (7) beim Aufbringen in einen nach außen verdeckten Aufnahmeraum (13; 22) des Verbindungsbereiches (4) angebracht wird.

2. Verfahren nach Anspruch 1, **dadurch gekennzeichnet, dass** vor dem Nachvernetzen ein Vorhärten der Silikonmasse (7) erfolgt.

3. Verfahren nach Anspruch 1 oder 2, **dadurch gekennzeichnet, dass** zum In-Kontakt-Bringen mindestens einer der Silikon-Schlauchkomponenten (3) mit der Silikon-Konnektorkomponente (2) im Verbindungsbereich (4) die beiden Komponenten (2, 3) ineinander geschoben werden.

4. Verfahren nach Anspruch 3, **dadurch gekennzeichnet, dass** beim Ineinanderschieben ein Vorzentrieren der Silikon-Schlauchkomponente (3) relativ zur Silikon-Konnektorkomponente (2) erfolgt.

5. Verfahren nach einem der Ansprüche 1 bis 4, **dadurch gekennzeichnet, dass** die Silikonmasse (7) über vordefinierte Einbringkanäle in den Aufnahmeraum (13; 22) eingebracht wird.

6. Verfahren nach Anspruch 1, **dadurch gekennzeichnet, dass** eine Sichtblende (12; 18) zum Verdecken des Aufnahmeraums (13) vor einem In-Kontakt-Bringen der mindestens einen Silikon-Schlauchkomponente (3) an der Silikon-Konnektorkomponente (2) aufgeweitet wird.

7. Fluidführungs-Baugruppe (11; 17; 19; 24)
- mit mindestens zwei fluidführenden Silikon-Schlauchkomponenten (3) und mindestens einer Silikon-Konnektorkomponente (2), zwischen denen eine fluidführende Verbindung über ein Verfahren nach einem der Ansprüche 1 bis 6 hergestellt ist.

8. Fluidführungs-Baugruppe nach Anspruch 7, **dadurch gekennzeichnet, dass** die Silikon-Schlauchkomponenten (3) und die Silikon-Konnektorkomponente (2) aus der gleichen Silikon-Materialzusammensetzung bestehen.

9. Fluidführungs-Baugruppe nach Anspruch 7 oder 8, **gekennzeichnet durch** eine Sichtblende (12; 18; 20) zum Verdecken mindestens eines Aufnahmeraums (13; 22) eines Verbindungsbereiches (4), an dem die Silikon-Schlauchkomponenten (3) an der Silikon-Konnektorkomponente (2) anliegen.

10. Fluidführungs-Baugruppe nach Anspruch 9, **dadurch gekennzeichnet, dass** die Sichtblende (12; 18) den Aufnahmeraum (13) vollständig nach außen abschließt.

11. Fluidführungs-Baugruppe nach Anspruch 9 oder 10, **gekennzeichnet durch** mindestens einen Einbringkanal in einer der Komponenten (2, 3) zum Einbringen der Silikonmasse (7) von außen durch diese Komponente (2, 3) in den Aufnahmeraum (13; 22).

12. Fluidführungs-Baugruppe nach einem der Ansprüche 7 bis 11, **gekennzeichnet durch** einen Zentrierabsatz (5; 9; 16) im Verbindungsbereich (4).

## Claims

1. Process for the manufacture of an impermeable connection between at least two fluid carrying silicone hose components (3) with the following steps:
- provision of the silicone hose components (3) and a silicone connector component (2);
- application of a fluid silicone mass (7) to at least one connecting area (4) where the silicone hose components (3) abut on the silicone connector component (2);
- polymeric post curing of the at least one connecting area (4) to harden the silicone mass (7),
**characterized in that**
- a contact between the silicone hose components (3) and the silicone connector component (2) is brought about before application of the fluid silicone mass (7), and
- during application, the silicone mass (7) is applied to an externally covered collection zone (13; 22) of the connecting area (4).

2. Process according to Claim 1, **characterized in that** a pre-hardening of the silicone mass (7) takes place prior to post curing.

3. Process according to Claims 1 or 2, **characterized in that** in order to bring at least one of the silicone hose components (3) into contact with the silicone connector component (2) in the connecting area (4), both components (2, 3) are pushed inside each other.

4. Process according to Claim 3, **characterized in that** the pushing of the components inside each other results in a pre-centering of the silicone hose component (3) relative to the silicone connector component (2).

5. Process according to one of Claims 1 to 4, **characterized in** the silicone mass is introduced into the collection zone (13; 22) through predefined introductory channels.

6. Process according to Claim 1, **characterized in that** a sheath (12; 18) to cover the collection zone (13) is expanded before the at least one silicone hose component (3) is brought into contact with the silicone connector component (2).

7. Fluid carrying assembly (11; 17; 19; 24)
- with at least two fluid carrying silicone hose components (3) and at least one silicone connector component (2) between which a fluid carrying connection is manufactured according to a method as claimed in any one of Claims 1 to 6.

8. Fluid carrying assembly according to Claim 7, **characterized in that** the silicone hose components (3) and the silicone connector component (2) are made from the same silicone material composition.

9. Fluid carrying assembly according to Claim 7 or 8, **characterized by** a sheath (12; 18; 20) to cover at least one collection zone (13; 22) of a connecting area (4) where the silicone hose components (3) abut on the silicone connector component (2).

10. Fluid carrying assembly according to Claim 9, **characterized in that** the sheath (12; 18) completely closes the collection zone (13) externally.

11. Fluid carrying assembly according to Claim 9 or 10, **characterized by** at least one introductory channel to one of the components (2, 3) for introduction of the silicone mass (7) in the collection zone (13; 22) externally through these components (2, 3).

12. Fluid carrying assembly according to one of Claims 7 to 11, **characterized by** a centering device (5; 9; 16) in the connecting area (4).

## Revendications

1. Procédé de fabrication d'une liaison étanche aux fluides entre au moins deux composants de tuyau en silicone (3) transportant des fluides, comprenant les étapes suivantes :
- mettant à disposition les composants de tuyau en silicone (3) et un composant connecteur en silicone (2),
- appliquant une masse de silicone (7) fluide sur au moins une zone de liaison (4) dans laquelle les composants de tuyau en silicone (3) sont placés contre le composant connecteur en silicone (2),
- soumettant l'au moins une zone de liaison (4) à une post-réticulation polymère pour durcir la masse de silicone (7),
**caractérisé en ce que**
- une mise en contact des composants de tuyau en silicone (3) sur le composant connecteur en silicone (2) est effectuée avant d'appliquer la masse de silicone (7) fluide et
- la masse de silicone (7), quand elle est appliquée, est introduite dans un espace de logement (13 ; 22) de la zone de liaison (4) qui est caché vers l'extérieur.

2. Procédé selon la revendication 1, **caractérisé en ce qu'**avant la post-réticulation, un prédurcissement de la masse de silicone (7) a lieu.

3. Procédé selon la revendication 1 ou 2, **caractérisé en ce que** pour la mise en contact d'au moins un des composants de tuyau en silicone (3) avec le composant connecteur de silicone (2) dans la zone de liaison (4), les deux composants (2, 3) sont glissés l'un dans l'autre.

4. Procédé selon la revendication 3, **caractérisé en ce qu'**en glissant les deux composants l'un dans l'autre, le composant de tuyau en silicone (3) est pré-centré relatif au composant connecteur en silicone (2).

5. Procédé selon l'une quelconque des revendications 1 à 4, **caractérisé en ce que** la masse de silicone (7) est introduite dans l'espace de logement (13 ; 22) via des canaux d'introduction prédéfinis.

6. Procédé selon la revendication 1, **caractérisé en ce qu'**une visière (12 ; 18) pour couvrir l'espace de logement (13) est évasée sur le composant connecteur en silicone (2) avant la mise en contact de l'au moins un composant de tuyau en silicone (3).

7. Module transportant des fluides (11 ; 17 ; 19 ; 24)
- comprenant au moins deux composants de tuyau en silicone (3) transportant des fluides et au moins un composant connecteur en silicone (2), entre lesquels une liaison transportant des fluides est établie par un procédé selon l'une quelconque des revendications 1 à 6.

8. Module transportant des fluides selon la revendication 7, **caractérisé en ce que** les composants de tuyau en silicone (3) et le composant connecteur en silicone (2) se composent de la même composition des matériaux en silicone.

9. Module transportant des fluides selon la revendication 7 ou 8, **caractérisé par** une visière (12 ; 18 ; 20) pour couvrir au moins un espace de logement (13 ; 22) d'une zone de liaison (4) dans laquelle les composants de tuyau en silicone (3) sont placés contre le composant connecteur en silicone (2).

10. Module transportant des fluides selon la revendication 9, **caractérisé en ce que** la visière (12 ; 18) couvre l'espace de logement (13) entièrement vers l'extérieur.

11. Module transportant des fluides selon la revendication 9 ou 10, **caractérisé par** au moins un canal d'introduction dans un des composants (2, 3) pour introduire la masse de silicone (7) de l'extérieur via ladite composant (2, 3) dans l'espace de logement (13 ; 22).

12. Module transportant des fluides selon l'une quelconque des revendications 7 à 11, **caractérisé par** un épaulement de centrage (5 ; 9 ; 16) dans la zone de liaison (4).
